# EUROPÄISCHE PATENTSCHRIFT

(11) **EP 1 428 858 B1**
(45) Veröffentlichungstag und Bekanntmachung des Hinweises auf die Patenterteilung: **30.01.2013**
(21) Anmeldenummer: 03028306.3
(22) Anmeldetag: 09.12.2003
(51) Int. Cl.: C09B 23/02, G01N 33/533, G01N 33/58

(54) **Hydrophile Marker auf der Basis von Benzopyrylo-Polymethinen**
Hydrophilic marker dyes based on benzopyrylo-polymethines
Colorants marqueurs hydrophiles à base de polyméthines de benzopyrylium

(30) Priorität: 10.12.2002 DE 10258150
(43) Veröffentlichungstag der Anmeldung: 16.06.2004
(73) Patentinhaber: Dyomics GmbH, 07745 Jena (DE)
(72) Erfinder: Czerney, Peter, 99425 Weimar (DE); Schweder, Bernd, 07743 Jena (DE); Wenzel, Matthias, 07749 Jena (DE); Frank, Wilhelm, 07743 Jena (DE)
(74) Vertreter: Oehmke, Volker

(56) Entgegenhaltungen:
- WO-A-00/53678
- WO-A-01/90253

## Beschreibung

Die Erfindung betrifft als Marker geeignete hydrophile Verbindungen auf der Basis von unsymmetrischen Polymethinen und ihre Verwendung in optischen, insbesondere fluoreszenzoptischen Bestimmungs- und Nachweisverfahren.

Typische Verfahrensanwendungen beruhen auf der Reaktion von farbstoffmarkierten Biomolekülen, wie zum Beispiel Antigenen, Antikörpern oder DNA-Segmenten mit der jeweils komplementären Spezies. Damit werden unter anderem Messungen von Enzymkinetiken, Rezeptor-Ligand-Interaktionen und Nucleinsäure-Hybridisierungskinetiken *in vitro* als auch *in vivo* ermöglicht. Des Weiteren sind die beanspruchten Marker für die pharmakologische Charakterisierung von Rezeptoren oder Wirkstoffen interessant.

Einsatzmöglichkeiten ergeben sich dadurch beispielsweise in der Medizin und Pharmazie, in der Bio- und Materialwissenschaft, bei der Umweltkontrolle und dem Nachweis von in der Natur und Technik vorkommenden organischen und anorganischen Mikroproben sowie anderes mehr.

Für diese Anwendungen werden üblicherweise symmetrische Xanthylium-Salze (Fluoresceine und Rhodamine) oder Polymethine (Indocyanine), wie sie beispielsweise in der US-Patentschrift 5 627 027 beansprucht werden, verwendet.

Alle diese Marker haben den Nachteil, dass sie aufgrund der Planarität des π-Elektronensystems zur Aggregation und Dimerenbildung, insbesondere in wässrigen Systemen neigen. Des Weiteren gehen ungenügend hydrophile Marker nicht spezifische Wechselwirkungen mit unterschiedlichen Oberflächen ein, was zu Problemen bei der Aufreinigung der entsprechenden Konjugate und zur einem nicht befriedigenden Signal/Rausch Verhältnis führt.

Um diese Nachteile zu umgehen, wurden in den Patentschriften WO 00 53 678 A und WO 01 90 253 A entsprechende nichtsymmetrische Polymethine auf der Basis von Benzo[*b*]pyran-2-yliden bzw. Benzo[*b*]pyran-4-yliden -Verbindungen beschrieben.

Uns gelang es nun, diese Marker durch die Einführung zusätzlicher, die Hydrophilie der Marker steigernde Substituenten weiter zu verbessern.

Gegenstand der Erfindung sind nun neue hydrophile Marker auf der Basis von Polymethinen der allgemeinen Formeln **I** und **II,** 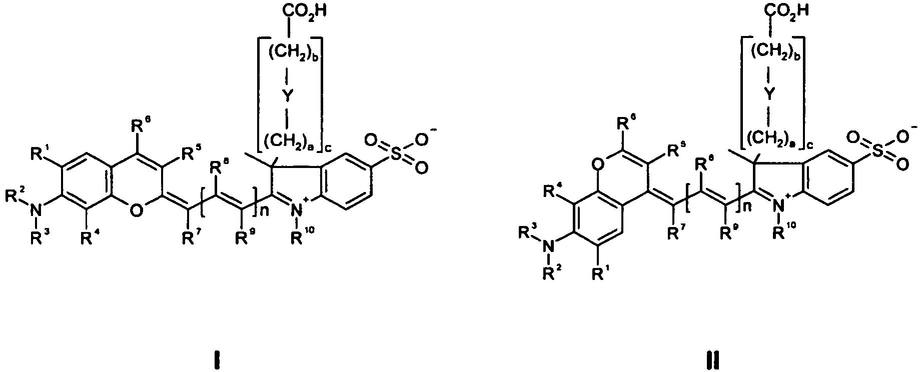 wobei
- R¹ - R¹⁰ gleich oder unterschiedlich sind und Wasserstoff, Alkyl-, tert-Alkyl, Aryl-, Carboxyaryl-, Dicarboxyaryl, Heteroaryl-, Cycloalkyl-, Heterocycloalkyl-, Alkyloxy-, Alkylmercapto- (wobei die Begriffe Alkyl und Cycloalkyl auch Reste mit olefinischen Bindungen einschließen sollen), Aryloxy-, Arylmercapto-, Heteroaryloxy-, Heteroarylmercapto-, Hydroxy-, Nitro- oder Cyano-Reste sein können und R¹ und R², R² und R³, R³ und R⁴, R⁵ und R⁷ bzw. R⁹ und R¹⁰ einen oder mehrere aliphatische, heteroaliphatische oder aromatische Ringe bilden können,
- einer oder mehrere der Substituenten R¹ - R¹⁰ solubilisierende bzw. ionisierbare bzw. ionisierte Substituenten wie SO₃⁻, PO₃²⁻, CO₂H, OH, NR₃⁺, Cyclodextrin oder Zucker darstellen, die die hydrophilen Eigenschaften der Farbstoffe
- bestimmen, wobei diese Substituenten auch über eine aliphatische oder heteroaliphatische, gegebenenfalls zyklische Spacergruppe am eigentlichen Grundchromophor angebunden sein können,
- mindestens einer der Substituenten R¹ - R¹⁰ bzw. die in 3-Position am Indolring befindliche Carboxyfunktion für eine reaktive Gruppe steht, welche eine kovalente Verknüpfung des Farbstoffs mit einem anderen Molekül ermöglicht, wobei dieser Substituent auch über eine Spacerfunktion am Markerfarbstoff angebunden sein kann,
- die reaktive Gruppe aus folgenden Funktionalitäten ausgewählt ist: Isocyanate, Isothiocyanate, Hydrazine, Amine, Mono- und Dichlor- bzw. Mono und Dibromtriazine, Aziridine, Sulfonylhalogenide, N-Hydroxysuccinimidester, Imido-Ester, Glyoxal oder Aldehyd für Amin- oder Hydroxy-Funktionen bzw. Maleimid oder lodacetamide für Thiol-Funktionen sowie Phosphoramidite für die Markierung von DNA oder RNA oder deren Bruchstücke und
- die aliphatische oder heteroaliphatische Spacergruppe aus einem Strukturelement -[(CH₂)ₐ-Y-(CH₂)_{b}]_{c}- besteht, worin Y gleich oder verschieden eine CR₂-, O-, S-, SO₂, SO₂NH-, NR-, COO- oder CONR- Funktion ist, wobei R die Funktionen von R¹ - R¹⁰ einnimmt und a und b gleich oder verschieden die Werte von 0 - 18 und c die Werte von 1- 18 darstellen,
- n für die Zahlenwerte 0, 1, 2 oder 3 steht, wobei die für n = 2 oder 3 jeweilig doppelt oder dreifach vorkommenden Substituenten R⁸ und R⁹ gleich oder unterschiedlich sein können,
- R⁶ einen Substituenten darstellt, der in α-oder γ-Position am Pyranring ein quartäres oder sp²-hybridisiertes C-Atom aufweist, wobei die Substituenten R⁶ und R⁵ auch ein aliphatisches bzw. substituiertes aliphatisches oder aromatisches Ringsystem bilden können.

Die erfindungsgemäßen Verbindungen können als Farbstoffe zur optischen Markierung von Proteinen, Nukleinsäuren, Oligomeren, DNA, RNA, biologischen Zellen, Lipiden, Mono-, Oligo- und Polysacchariden, Liganden, Rezeptoren, Polymeren, Pharmaka oder Polymerpartikeln verwendet werden und als Farbstoffe in Systemen zur qualitativen oder quantitativen Bestimmung von Proteinen, Nukleinsäuren, Oligomeren, DNA, RNA, biologischen Zellen, Lipiden, Polymeren, Pharmaka oder Polymerpartikeln über die funktionellen Gruppen an eine HO-, H₂N-, HS-oder HO₂C-Funktion der zu bestimmenden Substanzen gekoppelt werden.

Diese Kopplungsreaktion wird vorteilhaft in organischen oder wässrigen Lösungen durchgeführt.

Die aus den erfindungsgemäßen Verbindungen und Biomolekülen bestehenden Konjugate weisen fluoreszierende Eigenschaften auf bzw. deaktivieren den angeregten Zustand ohne Abgabe von Licht (Quencher).
Die erfindungsgemäßen Verbindungen finden in optischen, insbesondere fluoreszenzoptischen qualitativen und quantitativen Bestimmungsverfahren einschließlich Immuntests, Hybridisierungsverfahren, chromatographischen oder elektrophoretischen Verfahren, FRET-Systemen und Hoch-Durchsatz-Screenings oder zur Analyse von Rezeptor-Liganden-Wechselwirkungen auf einem Mikroarray Verwendung.

Die Polymethine der allgemeinen Formeln I und/oder 11 können als Farbstoffe zur optischen Markierung von organischen oder anorganischen Erkennungseinheiten, z. B. von Aminosäuren, Peptiden, Proteinen, Antigenen, Haptenen, Enzymsubstraten, Enzym-Cofaktoren, Biotin, Carotinoiden, Hormonen, Neurohormonen, Neurotransmittern, Wachstumsfaktoren, Lympholocinen, Lektinen, Toxinen, Kohlenhydraten, Oligosacchariden, Polysacchariden, Dextranen, Nucleinsäuren, Oligonucleotiden, DNA, RNA, biologischen Zellen, Lipiden, rezeptorbindenden Pharmaka oder organischen bzw. anorganischen polymeren Trägermaterialien verwendet werden.

Die Markierung der Erkennungseinheiten kann dabei durch die Ausbildung von ionischen Wechselwirkungen zwischen den Verbindungen der allgemeinen Formeln **I** und/oder **II** und den zu markierenden Materialien erfolgen.

Weiterhin besteht auch die Möglichkeit, die Erkennungseinheit oder das Trägermaterial kovalent mit dem Fluorophor zu verbinden. Diese Kopplungsreaktion kann in wässriger oder überwiegend wässriger Lösung und vorzugsweise bei Raumtemperatur durchgeführt werden. Dabei entsteht eine Sonde (Konjugat) zur qualitativen oder quantitativen Bestimmung von unterschiedlichen Biomaterialien bzw. anderen organischen und anorganischen Materialien unter Benutzung optischer Verfahren.

Sowohl die Verbindungen der allgemeinen **Formeln** I und/oder **II** und davon abgeleitete Systeme können in optischen, insbesondere fluoreszenzoptischen, qualitativen und quantitativen Bestimmungsverfahren zur Diagnostik von Zelleigenschaften (Molekulare Bildgebung), in Biosensoren (*point of care-*Messungen), zur Erforschung des Genoms und in Miniaturisierungstechnologien eingesetzt werden. Typische Anwendungen erfolgen in der Zytometrie und Zellsortierung, der Fluoreszenz-Korrelations-Spektroskopie (FCS), im *Ultra-High-Throughput-Screening* (UHTS), bei der *multicolor* Fluoreszenz-*in-situ*-Hybridisierung (FISH), in FRET-Systemen und in Mikroarrays (DNA- und Protein-Chips).

Dabei ist ein Mikroarray eine rasterartige Anordnung von auf mindestens einer Oberfläche immobilisierten Molekülen, die zum Studium von Rezeptor-Liganden-Wechselwirkungen verwendet werden können. Eine rasterartige Anordnung bedeutet mehr als zwei voneinander verschiedene Moleküle, welche sich innerhalb einer Fläche befinden und dort in unterschiedlichen, vorher definierten Regionen mit bekannter Position immobilisiert sind.

Ein Rezeptor ist ein Molekül, das eine Affinität zu einem gegebenen Liganden besitzt. Rezeptoren können natürlich vorkommende oder künstlich hergestellte Moleküle sein. Rezeptoren können in reiner Form oder gebunden an andere Spezies eingesetzt werden. Rezeptoren können kovalent oder nichtkovalent entweder direkt oder durch bestimmte Kopplungsvermittler an einen Bindungspartner angeknüpft werden.

Beispiele für Rezeptoren, die durch diese Erfindung detektiert werden können, schließen Agonisten und Antagonisten für Zell-Membran-Rezeptoren, Toxine und andere Giftstoffe, virale Epitope, Hormone wie Opiate und Steroide, Hormonrezeptoren, Peptide, Enzyme, Enzymsubstrate, als Kofaktoren agierende Wirkstoffe, Lektine, Zucker, Oligonukleotide, Nukleinsäuren, Oligosaccharide, Zellen, Zellfragmente, Gewebefragmente, Proteine und Antikörper ein, sind aber nicht auf die angeführten Stoffe beschränkt.

Ein Ligand ist ein Molekül, das von einem bestimmten Rezeptor erkannt wird. Beispiele für Liganden, die durch die erfindungsgemäßen Verbindungen detektiert werden können, schließen Agonisten und Antagonisten für Zell-Membran-Rezeptoren, Toxine und andere Giftstoffe, virale Epitope, Hormone wie Opiate und Steroide, Hormonrezeptoren, Peptide, Enzyme, Enzymsubstrate, als Kofaktoren agierende Wirkstoffe, Lektine, Zucker, Oligonukleotide, Nukleinsäuren, Oligosaccharide, Proteine und Antikörper ein, sind aber nicht auf die angeführten Stoffe beschränkt.

Durch die Darstellung von nichtsymmetrischen Polymethine, die einerseits als terminale Funktion einen leicht derivatisierbaren Heterocyclus vom Typ CH-acider Verbindungen, andererseits einen neuartig substituierten 6-Ringheterocyclus aufweisen, werden insbesondere nachfolgende Vorteile erreicht:

Bereits relativ kleine Moleküle absorbieren im Spektralbereich über 550 nm und zeigen gegenüber den bisher bekannten Polymethinen mit Absorptionsmaxima über 650 nm (Penta- und Heptamethine) eine wesentlich verbesserte photochemische und thermische Stabilität.

Durch *molecular engineering* ist es möglich, Lage und Intensität der Absorptions- und Emissionsmaxima beliebig zu steuern und den Emissionswellenlängen unterschiedlicher Anregungslaser, vor allem Diodenlasern, anzupassen.

Die erfindungsgemäßen Verbindungen sind relativ einfach durch das Kondensieren der beiden unterschiedlichen CH-aciden Heterocyclen und eines C-1, C-3 oder C-5 Baustein herzustellen (Eintopf-Verfahren).

Weitere Verfahren zu ihrer Herstellung bestehen darin, dass in einer 1. Reaktionsstufe einer der CH-aciden Heterocyclen mit dem C-1, C-3 oder C-5 Baustein kondensiert wird und nach Isolierung des 1:1-Kondensationsproduktes in einer nachfolgenden Kondensation mit dem zweiten CH-aciden Heterocyclus zum Polymethin umgesetzt wird. Dabei ist die Reihenfolge der Verwendung der Heterocyclen unerheblich. Dadurch ist in wenigen Reaktionsschritten eine Vielzahl unterschiedlich funktionalisierter, stark hydrophiler Farbstoffe, die sich hinsichtlich der Gesamtladung und der Spezifität/Reaktivität der zur Immobilisierung genutzten aktivierten Gruppen unterscheiden, auf einfache Weise herstellbar.

Die Erfindung soll nachstehend anhand von in der Zeichnung dargestellten Ausführungsbeispielen näher erläutert werden.

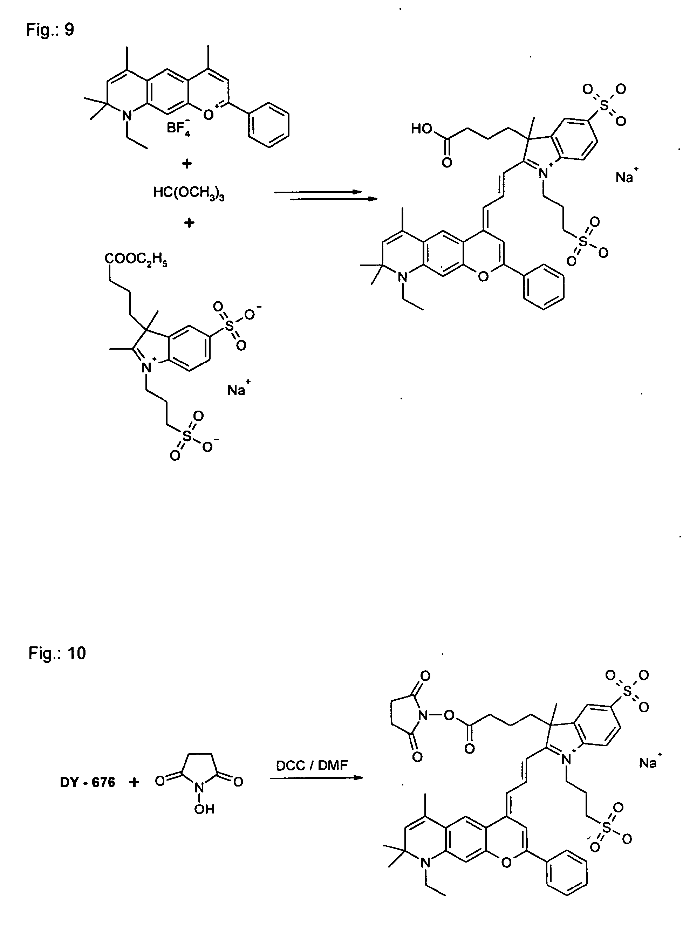

Es zeigen:

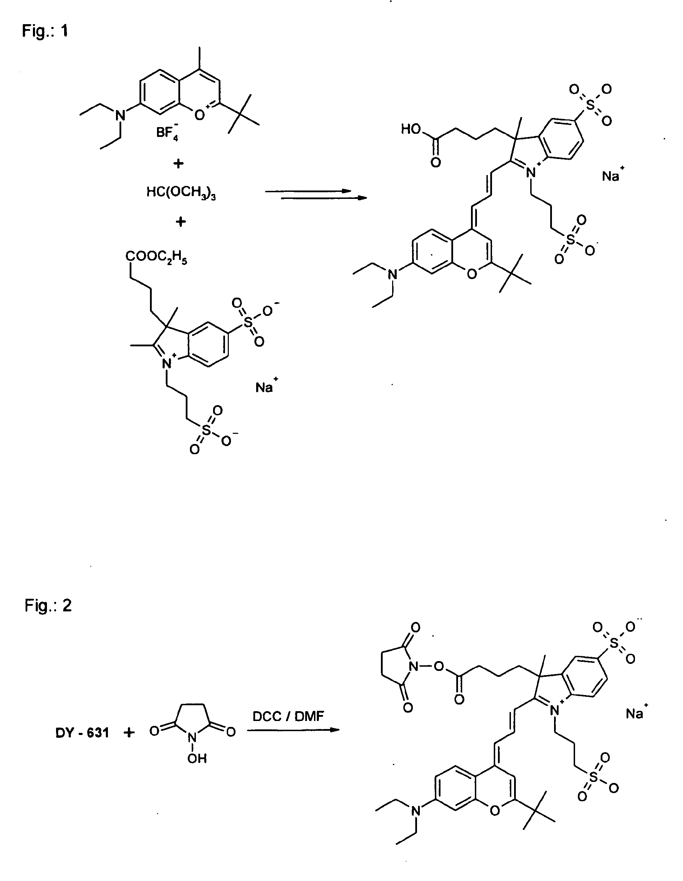

- Fig. 1:: Synthese von **DY - 631**
Ausführungsbeispiel **1**
- Fig. 2:: Synthese des N-Hydroxysuccinimid-Esters von **DY** - **631**
Ausführungsbeispiel **2**

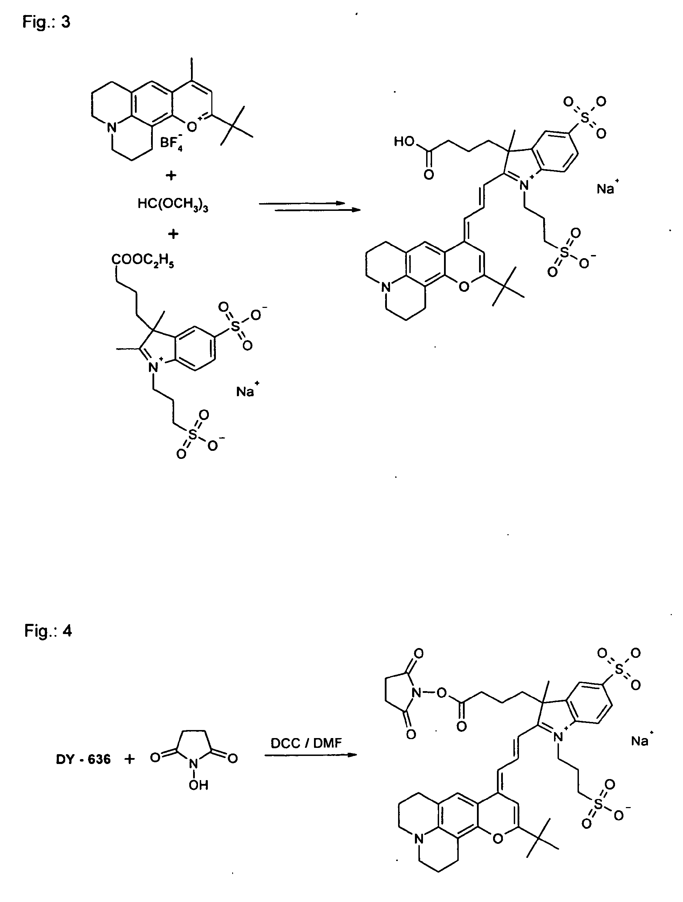

- Fig. 3:: Synthese von **DY - 636**
Ausführungsbeispiel **3**
- Fig. 4:: Synthese des N-Hydroxysuccinimid-Esters von **DY** - **636**
Ausführungsbeispiel **4**

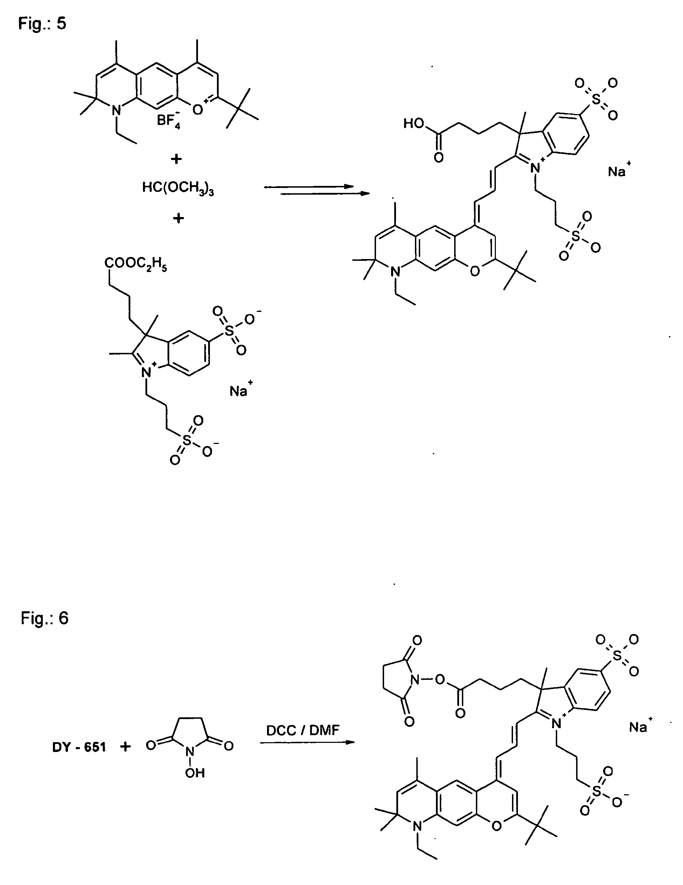

- Fig. 5:: Synthese von **DY - 651**
Ausführungsbeispiel **5**
- Fig. 6:: Synthese des N-Hydroxysuccinimid-Esters von **DY - 651**
Ausführungsbeispiel **6**

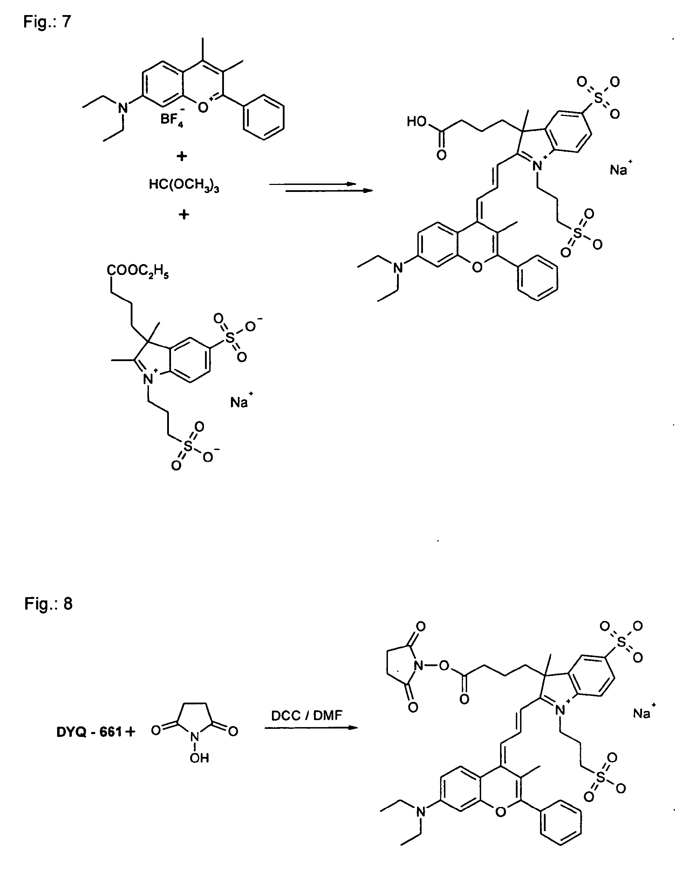

- Fig. 7:: Synthese von **DYQ - 661**
Ausführungsbeispiel **7**
- Fig. 8:: Synthese des N-Hydroxysuccinimid-Esters von **DYQ - 661**
Ausführungsbeispiel 8
- Fig. 9:: Synthese von **DY - 676**
Ausführungsbeispiel **9**
- Fig. 10:: Synthese des N-Hydroxysuccinimid-Esters von **DY - 676**
Ausführungsbeispiel **10**

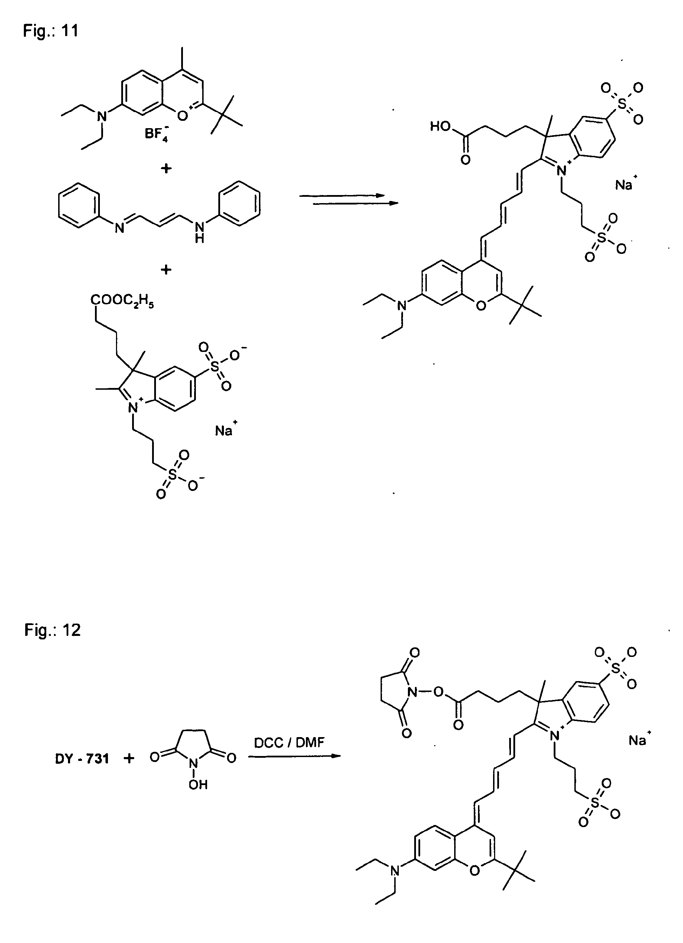

- Fig. 11:: Synthese von **DY - 731**
Ausführungsbeispiel **11**
- Fig. 12:: Synthese des N-Hydroxysuccinimid-Esters von **DY - 731**
Ausführungsbeispiel **12**

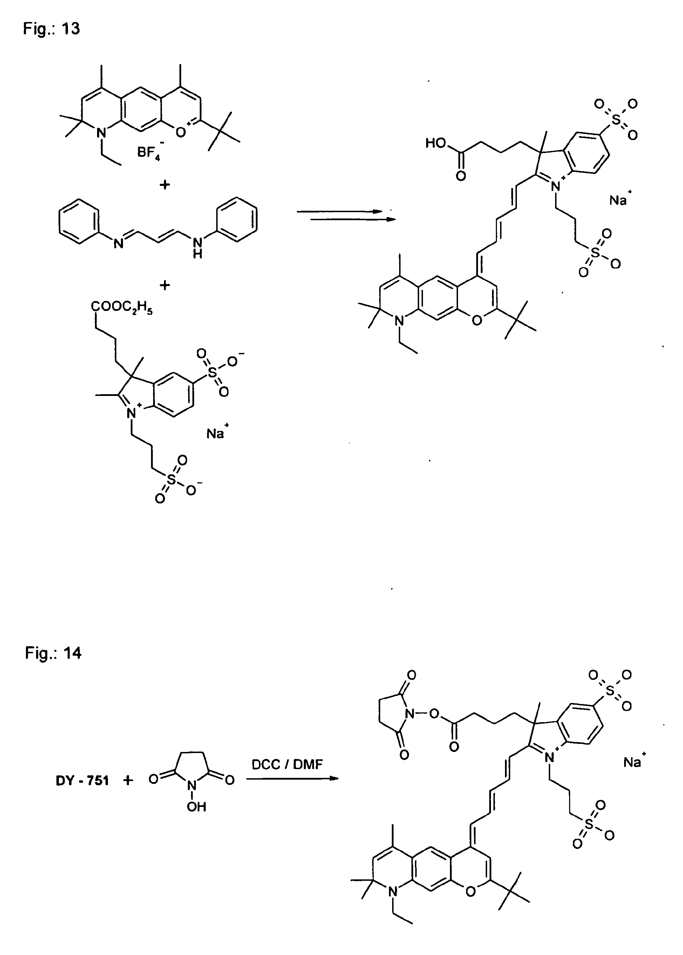

- Fig. 13:: Synthese von **DY - 751**
Ausführungsbeispiel **13**
- Fig. 14:: Synthese des N-Hydroxysuccinimid-Esters von **DY - 751**
Ausführungsbeispiel **14**

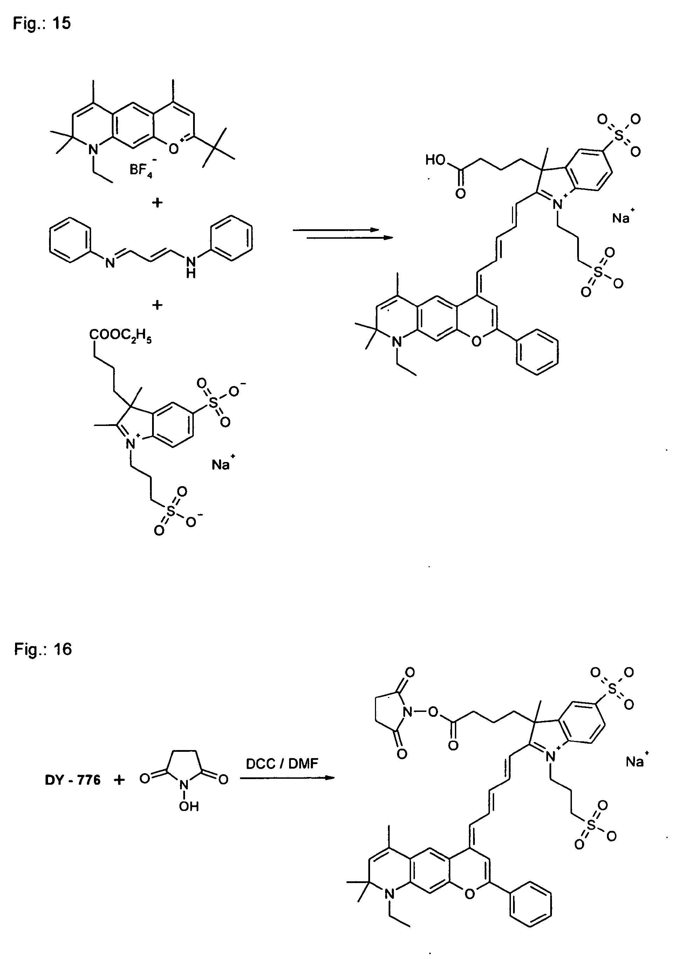

- Fig. 15:: Synthese von **DY - 776**
Ausführungsbeispiel **15**
- Fig. 16:: Synthese des N-Hydroxysuccinimid-Esters von **DY - 776**
Ausführungsbeispiel **16**

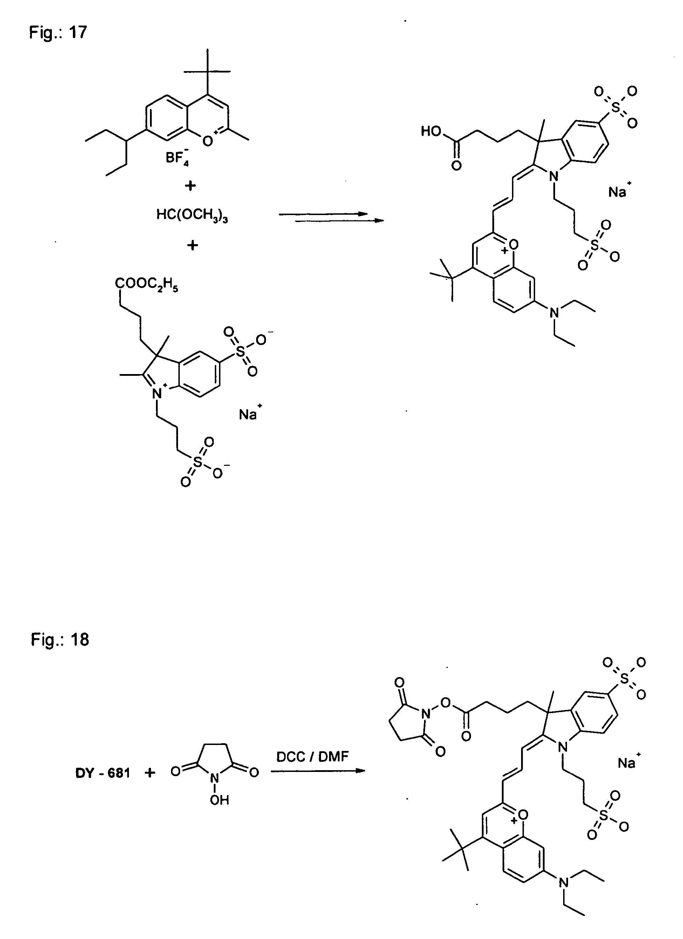

- Fig. 17:: Synthese von **DY - 681**
Ausführungsbeispiel **17**
- Fig. 18:: Synthese des N-Hydroxysuccinimid-Esters von **DY - 681**
Ausführungsbeispiel **18**

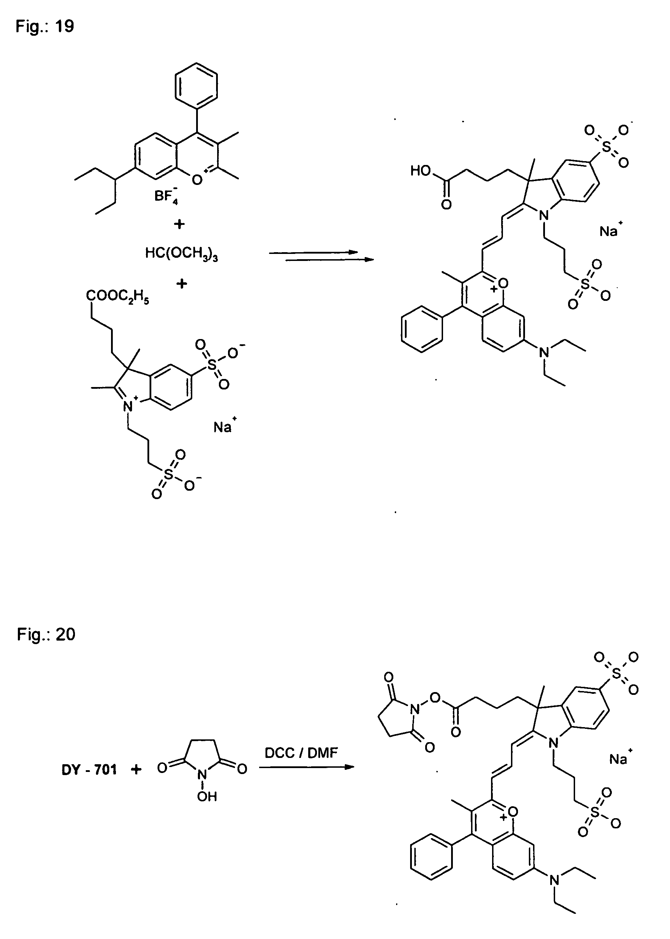

- Fig. 19:: Synthese von **DY - 701**
Ausführungsbeispiel **19**
- Fig. 20:: Synthese des N-Hydroxysuccinimid-Esters von **DY - 701**
Ausführungsbeispiel **20**

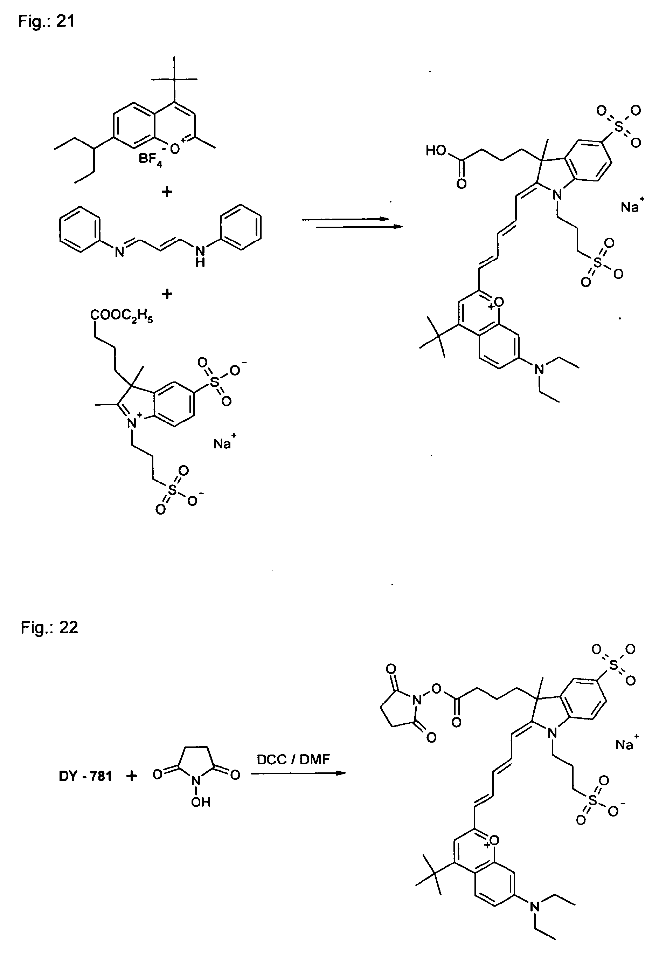

- Fig. 21:: Synthese von **DY - 781**
Ausführungsbeispiel **21**
- Fig. 22:: Synthese des N-Hydroxysuccinimid-Esters von **DY - 781**
Ausführungsbeispiel **22**

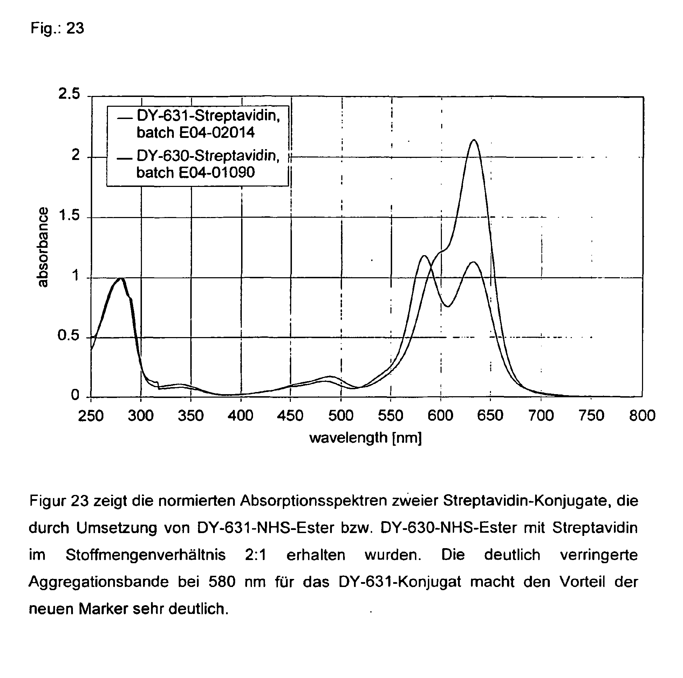

- Fig. 23:: UV-vis-Spektrum von DY-630- und DY-631-Streptavidin-Konjugat

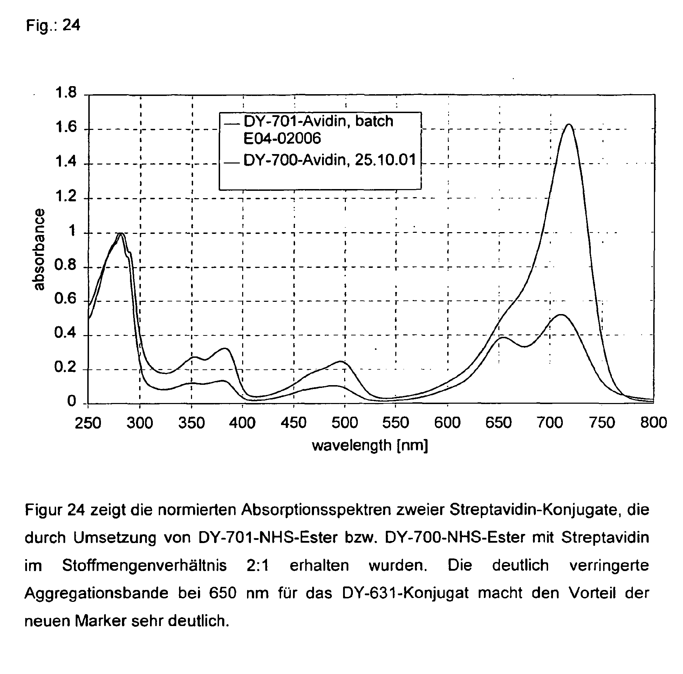

- Fig. 24:: UV-vis-Spektrum von DY-700- und DY-701-Avidin-Konjugat

Im folgenden bezieht sich der Begriff "Vakuum" auf den Druckbereich von 30 - 150 hPa. Die Mischungsverhältnisse von Flüssigkeiten sind Volumenverhältnisse. Und RT bedeutet Raumtemperatur.

### Ausführungsbeispiele 1 -16 (Verbindungen der allgemeinen Formel II)

### 1. Synthese von DY - 631

180 mg (0.5 mmol) 2-*tert*-Butyl-7-diethylamino-4-methyl-chromenylium-tetrafluoroborat und 242 mg (0.5 mmol) 3-(3-Ethoxycarbonylpropyl)-2,3-dimethyl-5-sulfonato-1-(3-sulfonatopropyl)-3*H*-indolium Natriumsalz werden in 50 ml Acetanhydrid gelöst, mit 75 µl (0,6 mmol) Orthoameisensäuretrimethylester und 1 ml Pyridin versetzt. Die Lösung wird bei ca. 140 °C ca. 30 min gerührt. Nach dem Abkühlen auf RT wird das Lösungsmittel im Vakuum abdestilliert.
Der Rückstand wird in einer Mischung aus 10 ml Aceton und 10 ml 2-molarer Salzsäure 2 Stunden unter Rückfluss erhitzt, die Reaktionslösung mit NaHCO₃ neutralisiert und das Lösungsmittel im Vakuum abdestilliert. Der Rückstand wird chromatographiert (SiO₂-RP-18, Eluent Methanol/ Wasser im Verhältnis 6:4).
145 mg (39 %) Ausbeute. - UV/Vis (Ethanol) λₘₐₓ (ε) = 637 nm (185.000 l·mol⁻¹·cm⁻¹). - Fluoreszenz λₑₘ = 658 nm. - MS (ESI⁻): 713.2 [M]⁻; 356.4 [M - H]²⁻. - **C₃₆H₄₅N₂O₉S₂Na** (736.88).

### 2. Synthese des N-Hydroxysuccinimid-Esters von DY - 631

15 mg **DY - 631,** 14 mg DCC und 4 mg NHS werden nach Ausführungsbeispiel 2 zur Reaktion gebracht und aufgearbeitet.

### 3. Synthese von DY - 636

206 mg (0.5 mmol) 10-*tert*-Butyl-8-methyl-2,3,5,6-tetrahydro-1*H*,4*H*-11-oxonia-3a-aza-benzo[de]anthracen-tetrafluoroborat und 242 mg (0.5 mmol) 3-(3-Ethoxy-carbonylpropyl)-2,3-dimethyl-5-sulfonato-1-(3-sulfonatopropyl)-3*H*-indolium Natriumsalz werden nach Ausführungsbeispiel 3 zur Reaktion gebracht und aufgearbeitet.
135 mg (36 %) Ausbeute. - UV/Vis (Ethanol) λₘₐₓ (ε) = 645 nm (155.000 l·mol⁻¹· cm⁻¹). - Fluoreszenz λₑₘ = 670 nm. - MS (ESI⁻): 737.1 [M]⁻; 368.4 [M-H]²⁻. - C₃₈H₄₅N₂O₉S₂Na (760.91).

### 4. Synthese des N-Hydroxysuccinimid-Esters von DY - 636

15 mg **DY - 636,** 14 mg DCC und 4 mg NHS werden nach Ausführungsbeispiel 2 zur Reaktion gebracht und aufgearbeitet.

### 5. Synthese von DY - 651

206 mg (0.5 mmol) 2-*tert*-Butyl-8-ethyl-4,5,7,7-tetramethyl-7,8-dihydro-1-oxonia-8-aza-anthracen-tetrafluoroborat und 242 mg (0.5 mmol) 3-(3-Ethoxycarbonylpropyl)-2,3-dimethyl-5-sulfonato-1-(3-sulfonatopropyl)-3*H*-indolium Natriumsalz werden nach Ausführungsbeispiel 3 zur Reaktion gebracht und aufgearbeitet.
145 mg (38 %) Ausbeute. - UV/Vis (Ethanol) λₘₐₓ (ε) = 653 nm (160.000 l·mol⁻¹· cm⁻¹). - Fluoreszenz λₑₘ = 678 nm. - MS (ESI⁻): 765.1 [M]⁻; 382.4 [M-H]²⁻. - C₄₀H₄₉N₂O₉S₂Na (888.96).

### 6. Synthese des N-Hydroxysuccinimid-Esters von DY - 651

15 mg **DY - 651,** 14 mg DCC und 4 mg NHS werden nach Ausführungsbeispiel 2 zur Reaktion gebracht und aufgearbeitet.

### 7. Synthese von DYQ - 661

196 mg (0.5 mmol) 7-Diethylamino-3,4-dimethyl-2-phenyl-chromenylium-tetrafluoroborat und 242 mg (0.5 mmol) 3-(3-Ethoxycarbonylpropyl)-2,3-dimethyl-5-sulfonato-1-(3-sulfonatopropyl)-3*H*-indolium Natriumsalz werden nach Ausführungsbeispiel 3 zur Reaktion gebracht und aufgearbeitet.
145 mg (37 %) Ausbeute. - UV/Vis (Ethanol) λₘₐₓ (ε) = 661 nm (116.000 l·mol⁻¹· cm⁻¹). - MS (ESI⁻): 747.1 [M]⁻, 373.6 [M - H]²⁻. - C₃₉H₄₃N₂O₉S₂Na (770.90).

### 8. Synthese des N-Hydroxysuccinimid-Esters von DYQ - 661

15 mg **DYQ - 661,** 14 mg DCC und 4 mg NHS werden nach Ausführungsbeispiel 2 zur Reaktion gebracht und aufgearbeitet.

### 9. Synthese von DY - 676

216 mg (0.5 mmol) 8-Ethyl-4,5,7,7-tetramethyl-2-phenyl-7,8-dihydro-1-oxonia-8-aza-anthracen-tetrafluoroborat und 242 mg (0.5 mmol) 3-(3-Ethoxycarbonylpropyl)-2,3-dimethyl-5-sulfonato-1-(3-sulfonatopropyl)-3*H*-indolium Natriumsalz werden nach Ausführungsbeispiel 3 zur Reaktion gebracht und aufgearbeitet.
150 mg (37 %) Ausbeute. - UV/Vis (Ethanol) λₘₐₓ (ε) = 674 nm (84.000 l·mol⁻¹· cm⁻¹). - Fluoreszenz λₑₘ = 699 nm. - MS (ESI⁺): 785.5 [M]⁺. - C₄₂H₄₅N₂O₉S₂Na (807.95).

### 10. Synthese des N-Hydroxysuccinimid-Esters von DY - 676

15 mg **DY - 676, 14** mg DCC und 4 mg NHS werden nach Ausführungsbeispiel 2 zur Reaktion gebracht und aufgearbeitet.

### 11. Synthese von DY - 731

180 mg (0.5 mmol) 2-*tert*-Butyl-7-diethylamino-4-methyl-chromenylium-tetrafluoroborat und 307 mg (0.5 mmol) 3-(3-Ethoxycarbonylpropyl)-3-methyl-2-(4-phenyl-aminobuta-1,3-dienyl)-5-sulfonato-1-(3-sulfonatopropyl)-3*H*-indolium Natriumsalz werden in 50 ml Acetanhydrid gelöst und mit 1 ml Pyridin versetzt. Die Lösung wird bei ca. 140 °C ca. 30 min gerührt. Nach dem Abkühlen auf RT wird das Lösungsmittel im Vakuum abdestilliert.

Der Rückstand wird in einer Mischung aus 10 ml Aceton und 10 ml 2-molarer Salzsäure 2 Stunden unter Rückfluss erhitzt, die Reaktionslösung mit NaHCO₃ neutralisiert und das Lösungsmittel im Vakuum abdestilliert. Der Rückstand wird chromatographiert (SiO₂-RP-18, Eluent Methanol/ Wasser im Verhältnis 6:4).
120 mg (31 %) Ausbeute. - UV/Vis (Ethanol) λₘₐₓ (ε) = 736 nm (225.000 l·mol⁻¹· cm⁻¹). - Fluoreszenz λₑₘ = 759 nm. - MS (ESI⁻): 739.2 [M]⁻. 369.5 [M - H]²⁻. - C₃₈H₄₇N₂O₉S₂Na (762.92).

### 12. Synthese des N-Hydroxysuccinimid-Esters von DY - 731

15 mg **DY - 731,** 14 mg DCC und 4 mg NHS werden nach Ausführungsbeispiel 2 zur Reaktion gebracht und aufgearbeitet.

### 13. Synthese von DY - 751

206 mg (0.5 mmol) 2-*tert*-Butyl-8-ethyl-4,5,7,7-tetramethyl-7,8-dihydro-1-oxonia-8-aza-anthracen-tetrafluoroborat und 307 mg (0.5 mmol) 3-(3-Ethoxycarbonylpropyl)-3-methyl-2-(4-phenyl-aminobuta-1,3-dienyl)-5-sulfonato-1-(3-sulfonatopropyl)-3*H-*indolium Natriumsalz werden nach Ausführungsbeispiel 15 zur Reaktion gebracht und aufgearbeitet.
120 mg (29 %) Ausbeute. - UV/Vis (Ethanol) λₘₐₓ (ε) = 751 nm (220.000 l·mol⁻¹· cm⁻¹). - Fluoreszenz λₑₘ = 779 nm. - MS (ESI⁺): 793.1 [M + H]⁺, 419.4 [M + 2 Na]²⁺, 408.4 [M + H + Na]²⁺, 397.4 [M + 2 H]²⁺. - C₄₂H₅₁N₂O₉S₂Na (814.99).

### 14. Synthese des N-Hydroxysuccinimid-Esters von DY - 751

15 mg **DY - 751**, 14 mg DCC und 4 mg NHS werden nach Ausführungsbeispiel 2 zur Reaktion gebracht und aufgearbeitet.

### 15. Synthese von DY - 776

216 mg (0.5 mmol) 8-Ethyl-4,5,7,7-tetramethyl-2-phenyl-7,8-dihydro-1-oxonia-8-aza-anthracen-tetrafluoroborat und 307 mg (0.5 mmol) 3-(3-Ethoxycarbonylpropyl)-3-methyl-2-(4-phenylamino-buta-1,3-dienyl)-5-sulfonato-1-(3-sulfonatopropyl)-3*H-*indolium Natriumsalz werden nach Ausführungsbeispiel 15 zur Reaktion gebracht und aufgearbeitet.
110 mg (26 %) Ausbeute. - UV/Vis (Ethanol) λₘₐₓ (ε) = 771 nm (147.000 l·mol⁻¹· cm⁻¹). - Fluoreszenz λₑₘ = 801 nm. - MS (ESI⁺): 813.1 [M + H]⁺, 429.2 [M + 2 Na]²⁺, 418.3 [M + H + Na]²⁺ , 407.3 [M + 2 H]²⁺. - C₄₄H₄₇N₂O₉S₂Na (834.98).

### 16. Synthese des N-Hydroxysuccinimid-Esters von DY - 776

15 mg **DY - 776,** 14 mg DCC und 4 mg NHS werden nach Ausführungsbeispiel 2 zur Reaktion gebracht und aufgearbeitet.

### Ausführungsbeispiele 17 - 22 (Verbindungen der allgemeinen Formel I)

### 17. Synthese von DY - 681

180 mg (0.5 mmol) 4-*tert*-Butyl-7-diethylamino-2-methyl-chromenylium-tetrafluoroborat und 242 mg (0.5 mmol) 3-(3-Ethoxycarbonylpropyl)-2,3-dimethyl-5-sulfonato-1-(3-sulfonatopropyl)-3*H*-indolium Natriumsalz werden nach Ausführungsbeispiel 3 zur Reaktion gebracht und aufgearbeitet.
140 mg (39 %) Ausbeute. - UV/Vis (Ethanol) λₘₐₓ (ε) = 691 nm (125.000 l·mol⁻¹· cm⁻¹). - Fluoreszenz λₑₘ = 708 nm. - MS (ESI⁻): 713.2 [M]⁻; 356.4 [M - H]²⁻. - C₃₆H₄₅N₂O₉S₂Na (736.88).

### 18. Synthese des N-Hydroxysuccinimid-Esters von DY - 681

15 mg **DY - 681,** 14 mg DCC und 4 mg NHS werden nach Ausführungsbeispiel 2 zur Reaktion gebracht und aufgearbeitet.

### 19. Synthese von DY - 701

196 mg (0.5 mmol) 7-Diethylamino-2,3-dimethyl-4-phenyl-chromenylium-tetrafluoroborat und 242 mg (0.5 mmol) 3-(3-Ethoxycarbonylpropyl)-2,3-dimethyl-5-sulfonato-1-(3-sulfonatopropyl)-3*H*-indolium Natriumsalz werden nach Ausführungsbeispiel 3 zur Reaktion gebracht und aufgearbeitet.
150 mg (39 %) Ausbeute. - UV/Vis (Ethanol) λₘₐₓ (ε) = 706 nm (115.000 l·mol⁻¹· cm⁻¹). - Fluoreszenz λₑₘ = 731 nm. - MS (ESI⁻): 747.2 [M]⁻; 373.4 [M-H]²⁻. - C₃₉H₄₃N₂O₉S₂Na (770.90).

### 20. Synthese des N-Hydroxysuccinimid-Esters von DY - 701

15 mg **DY - 701,** 14 mg DCC und 4 mg NHS werden nach Ausführungsbeispiel 2 zur Reaktion gebracht und aufgearbeitet.

### 21. Synthese von DY - 781

180 mg (0.5 mmol) 4-*tert*-Butyl-7-diethylamino-2-methyl-chromenylium-tetrafluoroborat und 307 mg (0.5 mmol) 3-(3-Ethoxycarbonylpropyl)-3-methyl-2-(4-phenyl-aminobuta-1,3-dienyl)-5-sulfonato-1-(3-sulfonatopropyl)-3*H*-indolium Natriumsalz werden nach Ausführungsbeispiel 15 zur Reaktion gebracht und aufgearbeitet.
125 mg (33 %) Ausbeute. - UVNis (Ethanol) λₘₐₓ (ε) = 783 nm (98.000 l·mol⁻¹· cm⁻¹). - Fluoreszenz λₑₘ = 800 nm. - MS (ESI⁺): 785.3 [M + Na]⁺; 763.3 [M + H]⁺; 404.4 [M + 2Na]²⁺; 393.5 [M + H +Na]²⁺. - C₃₈H₄₇N₂O₉S₂Na (762.92).

### 22. Synthese des N-Hydroxysuccinimid-Esters von DY - 781

15 mg **DY - 781,** 14 mg DCC und 4 mg NHS werden nach Ausführungsbeispiel 2 zur Reaktion gebracht und aufgearbeitet.

## Patentansprüche

1. Hydrophile Marker auf der Basis von unsymmetrischen Polymethinen der allgemeinen **Struktur I** bzw. **II** 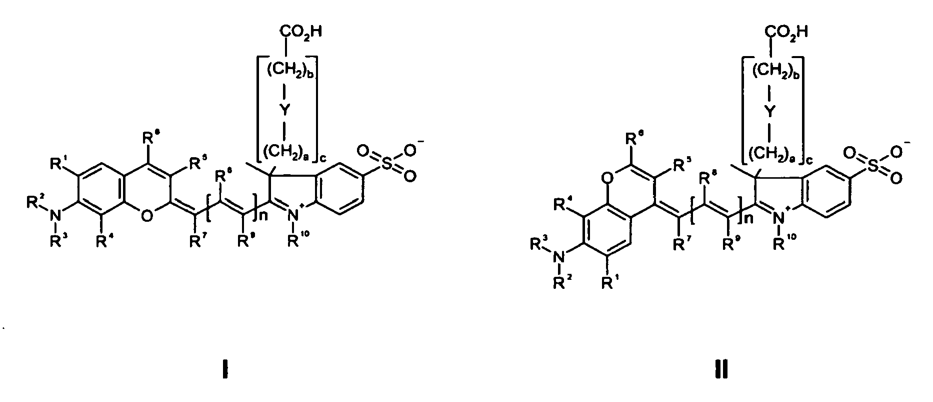 wobei
• n für die Zahlenwerte 0, 1, 2 oder 3 steht, wobei die für n = 2 oder 3 jeweilig doppelt oder dreifach vorkommenden Substituenten R⁸ und R⁹ gleich oder unterschiedlich sein können,
• R¹ - R¹⁰ gleich oder unterschiedlich sind und Wasserstoff, Alkyl-, *tert*-Alkyl, Aryl-, Carboxyaryl-, Dicarboxyaryl, Heteroaryl-, Cycloalkyl-, Heterocycloalkyl-, Alkyloxy-, Alkylmercapto- (wobei die Begriffe Alkyl und Cycloalkyl auch Reste mit olefinischen Bindungen einschließen sollen), Aryloxy-, Arylmercapto-, Heteroaryloxy-, Heteroarylmercapto-, Hydroxy-, Nitro- oder Cyano-Reste sein können und R¹ und R², R² und R³, R³ und R⁴, R⁵ und R⁷ bzw. R⁹ und R¹⁰ einen oder mehrere aliphatische, heteroaliphatische oder aromatische Ringe bilden können,
• mindestens einer der Substituenten R¹ - R¹⁰ solubilisierende bzw. ionisierbare bzw. ionisierte Substituenten wie SO₃⁻, PO₃²⁻, CO₂H, OH, NR₃⁺, Cyclodextrin oder Zucker darstellen, die die hydrophilen Eigenschaften der Farbstoffe bestimmen, wobei diese Substituenten auch über eine aliphatische oder heteroaliphatische, gegebenenfalls zyklische Spacergruppe am eigentlichen Grundchromophor angebunden sein können,
• mindestens einer der Substituenten R¹ - R¹⁰ bzw. die in 3-Position am Indolring befindliche Carboxyfunktion für eine reaktive Gruppe steht, welche eine kovalente Verknüpfung des Farbstoffs mit einem anderen Molekül ermöglicht, wobei dieser Substituent auch über eine Spacerfunktion am Markerfarbstoff angebunden sein kann,
• die reaktive Gruppe aus folgenden Funktionalitäten ausgewählt ist: Isocyanate, Isothiocyanate, Hydrazine, Amine, Mono- und Dichlor- bzw. Mono und Dibromtriazine, Aziridine, Sulfonylhalogenide, N-Hydroxysuccinimidester, Imido-Ester, Glyoxal oder Aldehyd für Amin- oder Hydroxy-Funktionen bzw. Maleimid oder lodacetamide für Thiol-Funktionen sowie Phosphoramidite für die Markierung von DNA oder RNA oder deren Bruchstücke und
• die aliphatische oder heteroaliphatische Spacergruppe aus einem Strukturelement -[(CH₂)ₐ-Y-(CH₂)_{b}]_{c}- besteht, worin Y gleich oder verschieden eine CR₂-, O-, S-, SO₂, SO₂NH-, NR-, COO- oder CONR- Funktion ist, wobei R die Funktionen von R¹ - R¹⁰ einnimmt und a und b gleich oder verschieden die Werte von 0 - 18 und c die Werte von 1 - 18 darstellen und
• R⁶ einen Substituenten darstellt, der in α- oder γ-Position am Pyranring ein quartäres oder sp²-hybridisiertes C-Atom aufweist, wobei die Substituenten R⁶ und R⁵ auch ein aliphatisches bzw. substituiertes aliphatisches oder aromatisches Ringsystem bilden können. heteroaliphatische, gegebenenfalls zyklische Spacergruppe am eigentlichen Grundchromophor angebunden sein können,
• mindestens einer der Substituenten R¹ - R¹⁰ bzw. die in 3-Position am Indolring befindliche Carboxyfunktion für eine reaktive Gruppe steht, welche eine kovalente Verknüpfung des Farbstoffs mit einem anderen Molekül ermöglicht, wobei dieser Substituent auch über eine Spacerfunktion am Markerfarbstoff angebunden sein kann, und
• die aliphatische oder heteroaliphatische Spacergruppe aus einem Strukturelement -[(CH₂)ₐ-Y-(CH₂)_{b}]_{c}- besteht, worin Y gleich oder verschieden eine CR₂-, O-, S-, SO₂, SO₂NH-, NR-, COO- oder CONR- Funktion ist, wobei R die Funktionen von R¹ - R¹⁰ einnehmen kann und a und b gleich oder verschieden die Werte von 0 - 18 und c die Werte von 1 - 18 darstellen und
• R⁶ einen Substituenten darstellt, der in α- oder γ-Position am Pyranring ein quartäres oder sp²-hybridisiertes C-Atom aufweist, wobei die Substituenten R⁶ und R⁵ auch ein aliphatisches bzw. substituiertes aliphatisches oder aromatisches Ringsystem bilden können.

2. Verwendung der hydrophilen Marker nach Anspruch 1 als Farbstoffe zur optischen Markierung von Aminosäuren, Proteinen, Antikörpern, Nucleinsäuren, Oligomeren, DNA, RNA, biologischen Zellen, Lipiden, Polymeren, Pharmaka oder Polymerpartikeln in optischen, insbesondere fluoreszenzoptischen qualitativen und quantitativen Bestimmungsverfahren einschließlich Immuntests, Hybridisierungsverfahren, FRET-Systemen, chromatographischer oder elektrophoretischer Verfahren und des Hoch-Durchsatz-Screenings.

3. Systeme zur qualitativen oder quantitativen Bestimmung von Aminosäuren, Proteinen, Antikörpern, Nucleinsäuren, Oligomeren, DNA, RNA, biologischen Zellen, Lipiden, Polymeren, Pharmaka oder Polymerpartikeln, **dadurch gekennzeichnet, dass** die funktionellen Gruppen der Verbindungen nach Anspruch 1 und 2 kovalent an eine HO-, H₂N-, oder HS-Funktion der zu bestimmenden Substanz gekoppelt werden.

4. Systeme nach Anspruch 3, **dadurch gekennzeichnet, dass** die Kopplungsreaktion in wässrigen Lösungen durchgeführt wird.

## Claims

1. Hydrophilic markers based on asymmetric polymethines of the general structure **I** or **II**: 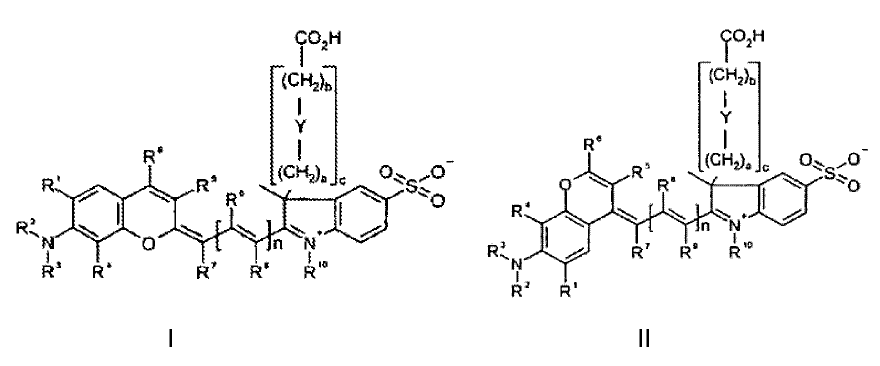 wherein
• n represents the numeric values 0, 1, 2, or 3, wherein the substituents R⁸ and R⁹, of which there are two or three each for each n = 2 or 3, respectively, may be identical or different;
• R¹-R¹⁰ are identical or different and may be hydrogen, alkyl, *tert*-alkyl, aryl, carboxyaryl, dicarboxyaryl, heteroaryl, cycloalkyl, heterocycloalkyl, alkyloxy, alkylmercapto (the terms alkyl and cycloalkyl also including radicals with olefinic bonds), aryloxy, arylmercapto, heteroaryloxy, heteroarylmercapto, hydroxy, nitro or cyano radicals, and R¹ and R², R² and R³, R³ and R⁴, R⁵ and R⁷ and/or R⁹ and R¹⁰ may form one or more aliphatic, heteroaliphatic or aromatic rings;
• at least one of the substituents R¹-R¹⁰ represents a solubilising or ionisable or ionised substituent, such as SO₃⁻, PO₃²⁻, CO₂H, OH, NR₃⁺, cyclodextrin or sugar, and determines the hydrophilic properties of the dyes, and said substituents may also be attached to the basic chromophore itself via an aliphatic or heteroaliphatic, optionally cyclic, spacer group;
• at least one of the substituents R¹-R¹⁰ and/or the carboxy function in 3-position on the indol ring may be a reactive group which enables covalent binding of the dye to another molecule, and said substituents may also be attached to the marker dye via a spacer group;
• the reactive group is selected from among the following functions: isocyanates, isothiocyanates, hydrazines, amines, monochloro triazines and dichloro triazines or monobromo triazines and dibromo triazines, aziridines, sulfonyl halides, *N*-hydroxysuccinimide esters, imidoesters, glyoxal or aldehyde for amino or hydroxy functions or maleimide or iodoacetamide for thiol functions as well as phosphoroamidites for marking DNA or RNA or fragments thereof, and
• the aliphatic or heteroaliphatic spacer group consists of a structural element -[(CH₂)ₐ-Y-(CH₂)_{b}]_{c}-, wherein Y is identical with or different from a CR₂-, O-, S-, SO₂, SO₂NH-, NR-, COO- or CONR function, wherein R may represent the functions of R¹-R¹⁰ and wherein a and b, which are identical or different, represent values from 0-18 and c represents values from 1-18, and
• R⁶ represents a substituent having a quarternary or sp²-hybridised C atom in α- or γ-position on the pyrane ring, and the substituents R⁶ and R⁵ may also form an aliphatic or substituted aliphatic or aromatic ring system.

2. Use of the hydrophilic markers according to claim 1 as dyes for the optical marking of amino acids, proteins, antibodies, nucleic acids, oligomers, DNA, RNA, biological cells, lipids, polymers, pharmaceuticals or polymer particles in optical, especially optical fluorescent, qualitative and quantitative determination processes, including immune tests, hybridisation procedures, FRET systems, chromatographic or electrophoretic procedures and high throughput screening.

3. Systems for qualitative or quantitative determination of amino acids, proteins, antibodies, nucleic acids, oligomers, DNA, RNA, biological cells, lipids, polymers, pharmaceuticals or polymer particles, **characterised in that** the functional groups of the compounds according to claims 1 and 2 are covalently coupled with an HO-, H₂N-, or HS- function of the substance to be determined.

4. The systems according to claim 3, **characterised in that** the coupling reaction is carried out in aqueous solutions.

## Revendications

1. Marqueurs hydrophiles à base de polyméthines asymmétriques de structure générale I ou II 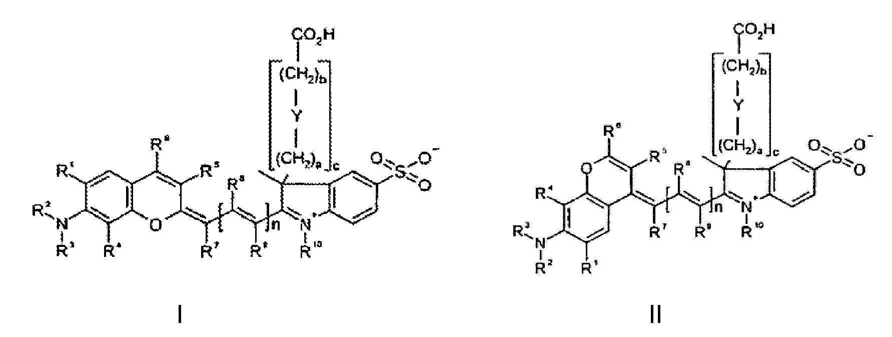 dans laquelle
• n représente les valeurs numériques 0, 1, 2, ou 3, et les substituants R⁸ et R⁹, dont il y a deux ou trois respectivement pour chaque n = 2 ou 3, peuvent être identiques ou différents;
• R¹-R¹⁰, identiques ou différents, peuvent être un atome d'hydrogène, un radical alkyle, tert-alkyle, aryle, carboxyaryle, dicarboxyaryle, hétéroaryle, cycloalkyle, hétérocycloalkyle, alkyloxy, alkylmercapto (alkyle et cycloalkyle comprenant également des radicaux avec des liaisons oléfiniques), aryloxy, arylmercapto, hétéroaryloxy, hétéroarylmercapto, hydroxy, nitro ou cyano, et R¹ et R², R² et R³, R³ et R⁴, R⁵ et R⁷ et/ou R⁹ et R¹⁰ peuvent former un ou plusieurs cycles aliphatiques, hétéroaliphatiques ou aromatiques;
• au moins un des substituants R¹-R¹⁰ est un substituant solubilisant ou ionisable ou ionisé, tel que SO₃⁻, PO₃²⁻, CO₂H, OH, NR₃⁺, cyclodextrine ou un sucre, qui détermine les propriétés hydrophiliques des colorants, et lesdits substituants peuvent également être liés au chromophore de base lui-même par le biais d'un groupe espaceur aliphatique ou hétéroaliphatique ou optionnellement cyclique;
• au moins un des substituants R¹-R¹⁰ et/ou la fonction carboxy en position 3 sur le cycle indole représente un groupe réactif permettant une liaison covalente du colorant à une autre molécule, ledit substituant pouvant également être lié au colorant de marquage par le biais d'un groupe espaceur;
• le groupe réactif est choisi parmi les fonctions suivantes: les isocyanates, les isothiocyanates, les hydrazines, les amines, les monochlorotriazines et les dichlorotriazines ou les monobromotriazines et les dibromotriazines, les aziridines, les halogénides d'acide sulfonique, les esters de N-hydroxysuccinimide, les imidoesters, le glyoxal ou aldéhyde pour les fonctions amino ou hydroxyle ou le maléimide ou les iodoacétamides pour les fonctions thioliques ainsi que les phosphoramidites pour le marquage d'ADN ou d'ARN ou de ses fragments, et
• le groupe espaceur aliphatique ou hétéroaliphatique consiste d'un élément structurel -[(CH₂)ₐ-Y-(CH₂)_{b}]_{c}-, dans lequel Y est identique avec ou différent d'une fonction CR₂-, O-, S-, SO₂, SO₂NH-, NR-, COO- ou CONR, où R représente les fonctions de R¹-R¹⁰, et dans lequel a et b, identiques ou différents, représentent les valeurs de 0-18, et c représente les valeurs de 1-18, et
• R⁶ représente un substituant présentant un atome de carbone quarternaire ou sp²-hybridisé en position α ou γ sur le cycle pyranique, les substituants R⁶ et R⁵ pouvant également former un système de cycle aliphatique ou aliphatique substitué ou aromatique.

2. Utilisation des marqueurs hydrophiles selon la revendication 1 comme colorants pour le marquage optique d'acides aminés, de protéines, d'anticorps, d'acides nucléiques, d'oligomères, d'ADN, D'ARN, de cellules biologiques, de lipides, de polymères, de médicaments ou de particules de polymère dans des procédés de détermination qualitatifs et quantitatifs optiques, en particulier optiques fluorescents, comprenant des tests immunologiques, des procédés d'hybridation, des systèmes FRET, des procédés chromatographiques ou électrophorétiques et de détection à rendement élevé.

3. Systèmes de détermination qualitative ou quantitative d'acides aminés, de protéines, d'anticorps, d'acides nucléiques, d'oligomères, d'ADN, D'ARN, de cellules biologiques, de lipides, de polymères, de médicaments ou de particules de polymère, **caractérisés en ce que** les groupes fonctionnels des composés selon les revendications 1 et 2 forment des liaisons covalentes avec une fonction HO-, H₂N-, ou HS- de la substance à déterminer.

4. Systèmes selon la revendication 3, **caractérisés en ce que** la réaction de liaison se produit dans des solutions aqueuses.
